(19) Europäisches Patentamt
European Patent Office
Office européen des brevets

(11) EP 4 206 217 A1

(12) EUROPEAN PATENT APPLICATION
published in accordance with Art. 153(4) EPC

(43) Date of publication:
**05.07.2023 Bulletin 2023/27**

(21) Application number: **21858402.7**

(22) Date of filing: **20.08.2021**

(51) International Patent Classification (IPC):
***C07K 14/165*** (2006.01) ***A61K 38/08*** (2019.01)
***A61K 38/10*** (2006.01) ***A61K 39/215*** (2006.01)
***A61P 31/14*** (2006.01) ***C07K 7/06*** (2006.01)
***C07K 7/08*** (2006.01) ***C12N 15/50*** (2006.01)

(52) Cooperative Patent Classification (CPC):
**A61K 38/08; A61K 38/10; A61K 39/215;
A61P 31/14; C07K 7/06; C07K 7/08; C07K 14/165**

(86) International application number:
**PCT/JP2021/030565**

(87) International publication number:
**WO 2022/039259 (24.02.2022 Gazette 2022/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.08.2020 JP 2020139794**
**08.12.2020 JP 2020203123**

(71) Applicant: **RIKEN**
**Wako-shi, Saitama 351-0198 (JP)**

(72) Inventors:
- **FUJII, Shinichiro**
  **Wako-shi, Saitama 351-0198 (JP)**
- **SHIMIZU, Kanako**
  **Wako-shi, Saitama 351-0198 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) METHOD FOR INDUCING IMMUNITY TO SARS-COV-2

(57) This disclosure relates to a peptide inducing immunity to SARS-CoV-2. Also, the disclosure relates to a composition containing the peptide and inducing immunity to SARS-CoV-2.

EP 4 206 217 A1

## Description

### Technical Field

The present invention relates to a method for inducing immunity to SARS-CoV-2, a peptide therefor and an immunogenic composition containing the peptide.

### Background Art

SARS-CoV-2 is a coronavirus that caused a pandemic in 2020. The World Health Organization (WHO) provisionally named the virus 2019-nCoV on January 7, 2020. On February 11 of the same year, the International Committee on Taxonomy of Viruses (ICTV) officially named the virus SARS-CoV-2. The WHO named a disease caused by the novel coronavirus, COVID-19. The ICTV specifies that SARS-CoV-2 belongs to the β coronavirus genus and the same species as SARS-CoV (or sister line). The complete genome sequence of SARS-CoV-2 has been registered in the National Center for Biotechnology Information (NCBI) of U.S., under GenBank registration (accession) number: MN908947.3.

The virus particles (virions) have a particle size of about 50 to 200 nm and contain a spike protein, a nucleocapsid protein, a membrane protein, an envelope protein and viral genome RNA, like a common coronavirus. The nucleocapsid protein forms a complex with RNA and a lipid-bound spike protein, a membrane protein and an envelope protein surround the complex to form the envelope of a virion. It is considered that the spike protein positioned on the outermost surface of the envelope binds to ACE2 receptor on the surface of a cell to promote transmission of a virus to the cell. There are persons, even if infected with SARS-CoV-2, develop no symptoms. Such persons refer to asymptomatic carriers. It is pointed out that asymptomatic carriers are likely to transmit the viruses they carry to others. It is also pointed out that persons infected with SARS-CoV-2 may have a decreased or absent sense of smell and/or taste. SARS-CoV-2 may cause severe acute respiratory syndrome. In severe acute respiratory syndrome, fever of about 40°C, cough and shortness of breath are reported as major symptoms. Pneumonia is mentioned as a significant complication.

Development of vaccines to SARS-CoV-2 has proceeded. In the current situation, it is desired to develop a wide variety of vaccines. For SARS-CoV-2, searches for a target peptide inducing an immune response have been conducted based on sequence homology and bioinformatic approach (Non Patent Literature 1).

### Citation List

#### Non Patent Literature

Non Patent Literature 1: Grifoni et al., Cell host & Microbe, 27(4): 671-680, 2020

### Summary of Invention

The present invention relates to a method for inducing immunity to SARS-CoV-2, a peptide therefor and an immunogenic composition containing the peptide.

More specifically, the present invention provides the following inventions.

(1) A partial peptide of SARS-CoV-2 spike protein comprising an amino acid sequence set forth in SEQ ID NO: 4 or a partial peptide of a spike protein positioned in correspondence with the sequence, and having an 8 to 30 amino acid length.
(2) The peptide according to (1), having an amino acid sequence of an 8 to 20 amino acid length within the region at positions 1204 to 1226 in an amino acid sequence set forth in SEQ ID NO: 8 or an amino acid sequence of a partial peptide of SARS-CoV-2 spike protein positioned in correspondence with the sequence.
(3) The peptide according to (1) or (2), consisting of the amino acid sequence set forth in SEQ ID NO: 4.
(4) The peptide according to (1) or (2), having an amino acid sequence set forth in any one of SEQ ID NOs: 10 to 12 and a 15 to 18 amino acid length.
(5) An immunogenic composition comprising the peptide according to any one of (1) to (4).
(6) A pharmaceutical composition comprising the peptide according to any one of (1) to (4) for use in inducing immunity to SARS-CoV-2.
(7) A method for inducing immunity to SARS-CoV-2 in a subject,
comprising administering the peptide according to any one of (1) to (4) or the composition according to (5) or (6) to the subject.
(8) The method according to (7), wherein the subject is a subject infected with SARS-CoV-2.

(9) The method according to (7), wherein the subject is a subject at risk of having been infected with SARS-CoV-2.
(10) The method according to (7), wherein the subject is a subject at risk of infection with SARS-CoV-2.

Brief Description of Drawings

[Figure 1] Figure 1 shows analysis of antigen-specific T cells obtained from PBMCs of 5 healthy donors (HD1 to HD5). Production of a cytokine by PBMCs was analyzed by FACS. After PBMCs were cultured for 21 days in the presence of each peptide, PBMCs were collected and evaluated for IFN-γ production. IFN-γ produced by T cells was evaluated by analysis based on intracellular staining after culture was carried out in the presence of GoldiPlug (BD) and each related peptide for 16 hours.
[Figure 2] Figure 2 shows cytotoxicity of T cells to HLA-A24+ target cells expressing Cov2-S antigen. A culture of PBMCs derived from HD5 was cultured in the presence of each peptide and a culture was collected three weeks later. The cytotoxicity of T cells to peptide-pulsed or non-pulsed HLA-A24+C1R was evaluated by co-culturing cytotoxic T cells (E) and HLA-A24+C1R cells (T) at a ratio (E/T) of 50: 1, 25: 1, or 12.5: 1.
[Figure 3] Figure 3 shows analysis of antigen-specific T cells obtained from PBMCs of 4 healthy volunteers (HV1 to 5). Production of cytokine by PBMCs was analyzed by FACS. After PBMCs were cultured for 21 days in the presence of each peptide, PBMCs were collected and evaluated for IFN-γ production. IFN-γ produced by T cells was evaluated by analysis based on intracellular staining after culture was carried out in the presence of GoldiPlug (BD) and each related peptide for 16 hours.
[Figure 4] Figure 4 shows a graph formed by plotting data of Figure 3.

Detailed Description of the invention

As used herein, a "subject" refers to a vertebrate, for example, a mammal including a human, and can be, e.g., a mammal (cat, ferret, bat and pangolin) to which SARS-CoV-2 is to be transmitted. A subject can be a subject infected with SARS-CoV-2, an asymptomatic carrier infected with SARS-CoV-2 and a subject who was infected with SARS-CoV-2 and has developed COVID-19. A subject can be a subject having a possibility (risk) that the subject might have been infected with SARS-CoV-2 or having a possibility (risk) that the subject may be infected with SARS-CoV-2. A subject can be a child (for example, an infant (1 year after birth to 6 years old), a schoolchild (6 to 12 years old), a youth (12 years old or older) or an adult (20 years old or older)). The adult can be 30 years old or older, 40 years old or older, 50 years old or older, 60 years old or older or 70 years old or older.
As used herein, "SARS-CoV-2" refers to a coronavirus that caused the pandemic of 2020. The World Health Organization (WHO) provisionally named the virus 2019-nCoV on January 7, 2020. On February 11 of the same year, the International Committee on Taxonomy of Viruses (ICTV) officially named the virus SARS-CoV-2. The coronavirus may cause common cold up to a serious respiratory disease such as severe acute respiratory syndrome (SARS) and middle east respiratory syndrome (MERS). The WHO named a disease caused by the novel coronavirus, COVID-19. The ICTV specifies that SARS-CoV-2 belongs to the β coronavirus genus and the same species as SARS-CoV (or sister line). The complete genome sequence of SARS-CoV-2 has been registered in the National Center for Biotechnology Information (NCBI) of U.S., under GenBank registration (accession) number: MN908947.3. Virus particles (virions) have a particle size of about 50 to 200 nm and contain a spike protein, a nucleocapsid protein, a membrane protein, an envelope protein and viral genome RNA, like a common coronavirus. The nucleocapsid protein forms a complex with RNA and a lipid-bound spike protein, a membrane protein and an envelope protein surround the complex to form the envelope of a virion. It is considered that the spike protein positioned on the outermost surface of the envelope binds to ACE2 receptor on the surface of a cell to promote transmission of a virus to the cell. There are persons, even if infected with SARS-CoV-2, develop no symptoms. Such persons refer to asymptomatic carriers. It is pointed out that asymptomatic carriers are likely to transmit the viruses they carry to others. It is also pointed out that persons infected with SARS-CoV-2 may have a decreased or absent sense of smell and/or taste. SARS-CoV-2 may cause severe acute respiratory syndrome. In severe acute respiratory syndrome, fever of about 40°C, cough and shortness of breath are reported as major symptoms. Pneumonia is mentioned as a significant complication. Whether or not a subject is infected with SARS-CoV-2 is primarily determined by a PCR test. In the PCR test, whether or not a gene of SARS-CoV-2 is present in the body is evaluated based on whether or not a band specific to SARS-CoV-2 is amplificated (appears). Examples of a treatment (drug) for SARS-CoV-2 include an antiviral drug to SARS-CoV-2 (for example, remdesivir), a steroidal anti-inflammatory drug (for example, dexamethasone) and an inflammatory cytokine inhibitor (for example, an IL-6 inhibitor, an anti-IL-6 antibody, a TNF-α inhibitor, an etanercept).
As used herein, a "spike protein" may be a protein encoded by nucleotides at positions 21563 to 25384 in SARS-CoV-2 genome, which is registered in the National Center for Biotechnology Information (NCBI) of U.S., under

GenBank registration number: MN908947.3. The SARS-CoV-2 spike protein has the amino acid sequence set forth in SEQ ID NO: 8 and also called as S protein.

As used herein, a "peptide" refers to an amino acid polymer. The polymer is basically not branched. A "partial peptide" refers to a part of a predetermined peptide. A peptide or a partial peptide thereof may bind to another protein, a lipid or an uncharged hydrophilic polymer (for example, polyethylene glycol) as long as it is exposed on a surface. The peptide or a partial peptide thereof can be produced based on a nucleic acid encoding the peptide. The peptide or a partial peptide thereof can be chemically synthesized. The peptide or a partial peptide thereof may be isolated, concentrated, or purified. Isolation means that the peptide or a partial peptide thereof is separated at least from other components. Purification means that the peptide or a partial peptide thereof is at least selectively separated. The concentration means that the concentration of the peptide or a partial peptide thereof is increased.

As used herein, a "composition" refers to a mixture of (1 or more) components. The composition may contain, for example, a partial peptide and an aqueous solvent (for example, water). The composition may further contain a pharmaceutical acceptable excipient. As used herein, an immunogenic composition refers to a composition that can induce an immune response in the body of a subject when it is administered to the subject. The immunogenic composition can be used for inducing an immune response in a subject. Since an immunogenic composition can induce an immune response in a subject, it can be used as a vaccine. The immunogenic composition of the present invention can be used for inducing an immune response to SARS-CoV-2 or used as a vaccine to SARS-CoV-2.

As used herein, "treatment" includes a preventive treatment and a therapeutic treatment. A treatment is applied to a symptomatic patient and(or) asymptomatic carrier. The therapeutic treatment is applied to transmitted virus, whereas the preventive treatment is applied in order to prevent future infection, delay onset of COVID-19 by future infection, or mitigate symptoms with COVID-19 after onset.

As used herein, a "human leukocyte antigen" (HLA) is a human major tissue compatibility complex (MHC). HLA is involved in presentation of an antigen to an immune cell and known to have various allyl polymorphisms. The allele that most frequently detected in Japanese people is A*24: 02 and about 36% of Japanese people has the allele. The peptide presented by HLA may be useful as a peptide vaccine; for example, a peptide presented by A*24: 02 may be useful as a peptide vaccine in Japanese people.

The disclosure provides a partial peptide of SARS-CoV-2 spike protein. The disclosure provides a partial peptide of SARS-CoV-2 spike protein, containing the amino acid sequence set forth in SEQ ID NO: 4 or a partial peptide of a spike protein (for example, a natural variant thereof) positioned in correspondence with the sequence.

In an embodiment, a spike protein of SARS-CoV-2 may be the spike protein that has been registered in the National Center for Biotechnology Information (NCBI) of U.S., under GenBank registration number: MN908947.3, or a spike protein of SARS-CoV-2 having an amino acid sequence identity of 90% or more, 910 or more, 92% or more, 93% or more, 94% or more, 95% or more, 96% or more, 97% or more, 98% or more or 99% or more, to that of the spike protein registered.

The disclosure provides a partial peptide of the SARS-CoV-2 spike protein containing the amino acid sequence set forth in SEQ ID NO: 4 or a partial peptide of a spike protein positioned in correspondence with the sequence, and having an 8 to 30 amino acid length (preferably, 8 to 10 amino acid length, 15 to 18 amino acid length, 8 to 20 amino acid length, 8 to 11 amino acid length or 9 amino acid length or 10 amino acid length and more preferably 9 amino acid length). Herein, the partial peptide having an amino acid sequence of a spike protein positioned in correspondence with the sequence refers to an amino acid sequence of a natural variant of SARS-CoV-2 spike protein that is arranged at a position corresponding to the SARS-CoV-2 spike protein when the variant protein is aligned with the SARS-CoV-2 spike protein.

The disclosure provides a peptide having a consecutive amino acid sequence of an 8 to 20 amino acid length (for example, 8 to 10 amino acid length, for example, 9 amino acid length or 15 to 18 amino acid length), within the region at positions 1204 to 1226 in the amino acid sequence set forth in SEQ ID NO: 8. The peptide herein contains the amino acid sequence set forth in SEQ ID NO: 4, according to a preferred embodiment.

The disclosure provides a composition containing two or more peptides selected from the group consisting of peptides having an amino acid sequence of an 8 to 20 amino acid length within the region at positions 1204 to 1226 in the amino acid sequence set forth in SEQ ID NO: 8 (for example, an 8 to 10 amino acid length, 9 amino acid length or 15 to 18 amino acid length). The composition, since it can provide various epitopes for TCR (T cell receptor) expressed on a T cell, can induce (activate) a T cell population having various antigen specificities.

A peptide of an 8 to 10 amino acid length (preferably 9 amino acid length) is directly presented by HLA class I (molecule) as an antigen and can activate a CD8 single-positive T cell. In contrast, a peptide of a 15 to 18 amino acid length is taken in a cell, degraded and presented by either one of HLA class I and II (molecule) as an antigen, and can activate a CD4 single-positive T cell and a CD8 single-positive T cell. A peptide of an 8 to 10 amino acid length (preferably 9 amino acid length) can be directly presented by HLA as an antigen not via a process of being taken and degraded in a cell, and presented by HLA as an antigen. Because of this, the peptide of an 8 to 10 amino acid length can quickly activate immunity. In contrast, a peptide of a 15 to 18 amino acid length can activate both CD4 single-positive T cell and

CD8 single-positive T cell. Those skilled in the art can select a peptide having an appropriate amino acid length depending on the purpose, and used as a vaccine.

According to this disclosure, there is provided T cells activated by the peptide of the present invention. According to the disclosure, there is provided a pharmaceutical composition containing T cells activated by the peptide of the present invention. The pharmaceutical composition (containing T cells activated by the peptide of the present invention) can be administered to a subject infected with a coronavirus such as SARS-CoV-2 and treat the infection in the subject. The pharmaceutical composition (containing T cells activated by the peptide of the present invention) has a condition for a solution suitably used for preparation of cells. The pharmaceutical composition (containing T cells activated by the peptide of the present inventio) may further contain a pharmaceutically acceptable excipient. Activation can be made by culturing a peptide, an antigen-presenting cell and T cells (for example, naive T cell, central memory T cell, effector memory T cell or terminal effector T cell). Culture herein can be carried out in the presence of a cytokine (for example, IL-2). T cells may be further activated in the presence of brefeldin A and/or monensin.

In the disclosure, a partial peptide of the SARS-CoV-2 spike protein may have a score of 0.1 or more, 0.2 or more, 0.3 or more, 0.4 or more, 0.5 or more, 0.6 or more or 0.7 or more according to NetMHCpan-4.0 (www.cbs.dtu.dk/services/NetMHCpan-4.0) by default setting (note that, A*24: 02).

In the disclosure, a partial peptide of the SARS-CoV-2 spike protein is, for example, a partial peptide of the SARS-CoV-2 spike protein containing the amino acid sequence set forth in SEQ ID NO: 4 or a partial peptide of a spike protein positioned in correspondence with the sequence. The partial peptide can be a peptide having an 8 to 30 amino acid length (preferably, 8 to 10 amino acid length, 15 to 18 amino acid length, 8 to 20 amino acid length, 8 to 11 amino acid length or 9 amino acid length or 10 amino acid length, more preferably 9 amino acid length) and having a score of 0.1 or more, 0.2 or more, 0.3 or more, 0.4 or more, 0.5 or more, 0.6 or more or 0.7 or more according to NetMHCpan-4.0 (www.cbs.dtu.dk/services/NetMHCpan-4.0) by default setting (note that, A*24: 02).

In the disclosure, a partial peptide of the SARS-CoV-2 spike protein is, for example, a partial peptide of the SARS-CoV-2 spike protein having an amino acid sequence identity of 90% or more, 910 or more, 92% or more, 93% or more, 94% or more, 95% or more, 96% or more, 97% or more, 98% or more or 99% or more, to the amino acid sequence set forth in SEQ ID NO: 4, and having a score of 0.1 or more, 0.2 or more, 0.3 or more, 0.4 or more, 0.5 or more, 0.6 or more or 0.7 or more according to NetMHCpan-4.0 (www.cbs.dtu.dk/services/NetMHCpan-4.0) by default setting (note that, A*24: 02).

According to the disclosure, the peptide can be satisfactorily presented by HLA as an antigen in a human having HLA of, for example, A*24: 02. An immune response specific to the peptide can be induced by the antigen presentation. Accordingly, the peptide according to the disclosure can be used for inducing an immune response specific to the peptide.

The peptide according to the disclosure can induce T cell immunity specific to the peptide. Accordingly, the peptide according to the disclosure can be used for inducing T cell immunity specific to the peptide.

The peptide according to the disclosure can induce immune response to SARS-CoV-2. Accordingly, the peptide according to the disclosure can be used for inducing an immune response to SARS-CoV-2.

The peptide according to the disclosure can induce T cell immunity to SARS-CoV-2. Accordingly, the peptide according to the disclosure can be used for inducing T cell immunity to SARS-CoV-2.

The peptide according to the disclosure can be used as a vaccine to SARS-CoV-2.

The peptide according to the disclosure can be contained in an immunogenic composition. Accordingly, the disclosure provides an immunogenic composition containing the peptide according to the disclosure. The immunogenic composition may further contain an immune adjuvant (adjuvant). Examples of the adjuvant include a settable adjuvant and an oily adjuvant. Examples of the settable adjuvant include sodium hydroxide, aluminum compound (adjuvant) such as aluminum potassium sulfate, aluminum phosphate, aluminum hydroxide, calcium phosphate, alum, HEPES and a carboxyvinyl polymer. Examples of the oily adjuvant include liquid paraffin, lanolin and Freund's adjuvant. Examples of the (Freund's) adjuvant include incomplete Freund's adjuvant and complete Freund's adjuvant. Examples of the other adjuvants that can be used include a Toll like receptor (TLR) ligand and an antigen-presenting cell. The TLR ligand is a molecule binding to at least one TLR to activate the TLR, more specifically, activate TLR mediated cell signaling. Examples of the TLR ligand may include TLR1 ligand, TLR2 ligand, TLR3 ligand, TLR4 ligand, TLR5 ligand, TLR6 ligand, TLR7 ligand, TLR8 ligand or TLR9 ligand; TLR7 agonist, TLR8 agonist or TLR9 agonist. Specific examples thereof include double strand RNA, muramyl dipeptide (MDP), threonyl-muramyl dipeptide (t-MDP), OM-174, flagellin, single strand RNA, oligoribonucleotide (ORN), imidazoquinoline amine (for example, imiquimod (R-837)), lipopolysaccharide (LPS) and a derivative thereof (for example, triacylated lipopeptide such as Pam3CSK4; and lipoglycan such as lipoarabinomannan and lipomannan), zymosan, monophospholipid A, FSL-1, loxoribine, bropirimine, ss poly U, resiquimod (R-848), CpG oligodeoxynucleotide (CpG-ODN), CpG pathogen-associated molecular patterns (PAMP), polyinosinic acid polycytidylic acid (PolyIC), polyinosinic acid-polycytidylic acid-poly L-lysine (Poly-ICLC), a nucleic acid containing unmethylated CpG island, DNA derived from a virus or bacterium, a phosphorothioate-containing molecule (for example, phosphorothioate nucleotide analogue), a nucleic acid having a phosphorothioate backbone, STING ligand and granzyme A. Examples of the antigen-presenting cell include dendritic cell, macrophage, and B cell. In an embodiment, the immunogenic composition is administered to a human.

An immunogenic composition is aseptic. In an embodiment, an immunogenic composition does not contain a pyrogen. An immunogenic composition is adjusted to have pH6 to 8 (for example, pH7 to 7.4). An immunogenic composition can be formulated into an injectable preparation. An immunogenic composition can be formulated into a preparation for inhalation administration. An immunogenic composition may further contain a pharmaceutically acceptable excipient. Examples of the pharmaceutically acceptable excipient include water, saline, a pH buffer, a tonicity agent, a preservative and an antioxidant. An immunogenic composition can be administered, for example, parenterally, e.g., intramuscularly or intravenously.

According to the disclosure, there is provided a method for inducing antigen-specific immunity in a subject, including administering an effective amount of the peptide or immunogenic composition according to the disclosure, to the subject. According to the disclosure, there is provided a method for inducing antigen-specific T-cell immunity in a subject, including administering an effective amount of the peptide or immunogenic composition according to the disclosure, to the subject. According to the disclosure, there is provided a method for inducing antigen-specific cytotoxic T cells in a subject, including administering an effective amount of the peptide or immunogenic composition according to the disclosure, to the subject. According to the disclosure, there is provided a method for inducing immunity to SARS-CoV-2 in a subject, including administering an effective amount of the peptide or immunogenic composition according to the disclosure, to the subject.

According to the disclosure, there is provided use of the peptide or immunogenic composition disclosed herein in a subject for manufacturing a medicament for use in inducing antigen-specific immunity in the subject. According to the disclosure, there is provided use of the peptide or immunogenic composition disclosed herein in a subject for manufacturing a medicament for use in inducing antigen-specific T cell immunity in the subject. According to the disclosure, there is provided use of the peptide or immunogenic composition disclosed herein in a subject for manufacturing a medicament for use in inducing antigen-specific cytotoxic T cells in the subject. According to the disclosure, there is provided use of the peptide or immunogenic composition disclosed herein in a subject for manufacturing a medicament for use in inducing immunity to SARS-CoV-2 in the subject.

Whether or not antigen-specific immunity was induced in a subject can be determined based on an increase of INF-$\gamma$ production in cytotoxic T cells present in peripheral blood of a subject. In the case where INF-$\gamma$ production in cytotoxic T cells significantly increased, it can be determined that antigen-specific immunity was induced in a subject. INF-$\gamma$ production in cytotoxic T cells compared to that before administration can be, for example, 1.1 times or more, 1.2 times or more, 1.3 times or more, 1.4 times or more, 1.5 times or more, 1.6 times or more, 1.7 times or more, 1.8 times or more, 1.9 times or more, 2 times or more, 3 times or more, 4 times or more, 5 times or more, 6 times or more, 7 times or more, 8 times or more, 9 times or more or 10 times or more. Accordingly, in the case where INF-$\gamma$ production in cytotoxic T cells increased at a rate as mentioned above, it can be determined that antigen-specific immunity was induced in a subject. Cytotoxic T cells, after they are cultured in the presence of IL-2, can be subjected to measurement for determination as mentioned above. Cytotoxic T cells can be isolated by flow cytometry known to those skilled in the art. Cytotoxic T cells can be isolated based on expression of, for example, CD3 and CD8, as an index.

According to the disclosure, there is provided a T cell receptor (TCR) containing TRA ($\alpha$ chain) according to any one of No. 1 to No. 12 in Table 3 and TRB ($\beta$ chain) according to any one of No. 1 to No. 12 in Table 3. According to the disclosure, there is provided TCR containing TRA and TRB according to any one of No. 1 to No. 12 in Table 3. According to the disclosure, there is provided a T cell receptor (TCR) containing TRA according to any one of No. 1 to No. 21 in Table 4 and TRB according to any one of No. 1 to No. 21 in Table 4. According to the disclosure, there is provided TCR containing TRA and TRB according to any one of No. 1 to No. 21 in Table 4.

According to the disclosure, there is provided a cell, on the surface of which at least one TCR selected from the groups consisting of TCRs mentioned above is expressed. According to the disclosure, there is provided a pharmaceutical composition containing a cell, on the surface of which at least one TCR selected from the groups consisting of TCRs mentioned above is expressed. The cell can be, for example, an immune cell, which can be at least one cell selected from the group consisting of e.g., T cell, NK cell, NKT cell, $\gamma\delta$ T cell and MAIT cell. The pharmaceutical composition can be used for treating an infection with a coronavirus such as SARS-CoV-2. According to the disclosure, there is provided a T cell (for example, T cell producing INF-$\gamma$ or TNF-$\alpha$) to be activated in response to the above peptide. Such a T cell can be induced by at least one of the peptides mentioned above. According to the disclosure, there is provided a pharmaceutical composition containing a T cell to be activated in response to the above peptide. These pharmaceutical compositions can be used for treating an infection with a coronavirus such as SARS-CoV-2. According to the disclosure, there is provided a pharmaceutical composition containing a cell (particularly, immune cell) having TCR to be activated in response to the above peptide. These pharmaceutical compositions can be used for treating an infection with a coronavirus such as SARS-CoV-2.

The peptide or immunogenic composition according to the disclosure can be administered to a subject once or a plurality of times, at a frequency of, for example, once per week.

Examples

Example 1: Synthesis of a peptide and induction of immune to the peptide

1. Materials and methods

1.1 Specimen of a human subject and preparation thereof

PBMCs were taken from healthy 5 donors (HD1 to HD5) belonging to RIKEN and separated by Ficoll-paque™ PLUS (GE Healthcare) density-gradient centrifugation. The PBMCs were washed twice with PBS and kept in liquid nitrogen until use.

1.2. Reagents and antibody

Human IL-2 (hIL-2) was purchased from Shionogi & Co., Ltd. The following monoclonal antibodies (mAb) were purchased from Biolegend: anti-human mAbs; PE/Cy7 conjugated anti CD3, PerCP-Cy5.5 conjugated anti CD4, FITC conjugated anti CD8, APC conjugated anti IFN-g mAbs. Topo-Pro-3 and a carboxyfluorescein succinimidyl ester (CFSE) were purchased from Thermo Fisher Scientific.

1.3. T cell culture

PBMCs were cultured in RPMI containing 10% FBS and 100 U/ml hIL-2 and stimulated with each of the peptides (10 μM, GeneScript) listed in Table 1 (below). Restimulation with the same peptide was repeated once per week for 21 days. It is considered that T cells having binding affinity to a peptide can survive by stimulation with the peptide. The peptides listed in Table 1 are estimated as HLA-A*24: 02-restrictive peptides present in the nucleocapsid or spike protein of SARS-CoV-2. Estimation was carried out by use of NetMHCpan-4.0. The peptides of Table 1 are designed so as to have an 8 to 10 amino acid length and allow CD8 single-positive T cells to survive when they bound to HLA class I and stimulated T cells.

[Table 1]

Table 1: Amino acid sequences of peptides used herein and their origins

| SEQ ID NO/ peptide No. (pep#) | Amino acid sequence |
|---|---|
| 1 | N F K D Q V I L L (Nucleocapsid protein) |
| 2 | V Y S T G S N V F (Spike protein) |
| 3 | V Y S S A N N C T F (Spike protein) |
| 4 | Q Y I K W P W Y I (Spike protein) |
| 5 | Y N Y L Y R L F (Spike protein) |
| 6 | Y F P L Q S Y G F (Spike protein) |
| 7 | T Y V P A Q E K N F (Spike protein) |

1.4. Flow cytometry

IFN-γ cannot be produced just simply by stimulating T cells with a peptide as mentioned above. Then, for intracellular staining of INF-γ, cells were cultured in the presence or absence of each peptide for 16 hours, together with brefeldin A and monensin. In this manner, INF-γ production from T cells was induced. By the experimental operation, T cells having a peptide-specific TCR conceivably produce INF-γ. After the surface of the cells was stained with surface antigen CD3, CD4 and CD8, the cells were fixed and permeabilized by BD Cytofix/Cytoperm™ (BD) for staining intracellular cytokines. Data were analyzed by FlowJo software (Tree Star).

1.5. Cytotoxicity assay

Three weeks later, cultured CTL strain was collected as effector cells. T-cell cytotoxicity assay was carried out by use of the following target cells. A24-CIR cells labeled with CFSE were pulsed with a peptide instructed or DMSO for 2 hours and washed twice. The effector cells were cultured together with $1 \times 10^4$ targets cells for 6 hours so as to satisfy a ratio of effector cells/target cells of 12.5, 25, and 50. Six hours later and immediately before analysis, the cells were stained with To-PRO3 to distinguish dead cells. Spontaneous death (SD) of target cells was determined based on the label attached to target cells cultured alone. As a positive control for total cytotoxicity (TD), labeled target cells were permeabilized by BD Cytofix/Cytoperm reagent (BD Pharmingent). Specific dissolution was calculated in accordance with the following calculation formula:

$$(\text{Sample-SD/TD-SD}) \times 100.$$

The cells were analyzed by flow cytometry.

2. Results

Whether or not expression of intracellular TNF-γ in CD8 single-positive T cells is induced by each of the antigen peptides listed in Table 1 was confirmed by flow cytometry. As a negative control, PBMCs not stimulated with a peptide were used. Data were compared between the case where stimulation was made by a peptide and the case where no stimulation was made by the peptide, per every healthy donor (HD1 to HD5). The results were as shown in Figure 1. As shown in Figure 1, pet#4 peptide exhibited strong induction of IFN-γ production to (in) CD8 single-positive T cells (cytotoxic T cells).

PBMCs taken from healthy donor HD-5 were pulsed with pep#4 peptide in the presence of IL-2 and a culture of PBMCs was obtained. Thereafter, cytotoxic T cells (CTL) were isolated from the culture of PBMC obtained. A24-CIR cells labeled with CFSE were pulsed with pep#4 peptide to allow HLA on the C1R cell surface to present pep#4 peptide. Thereafter, the CTL was allowed to act on the A24-C1R cells obtained to confirm whether antigen-specific CTLs were induced or not. As a negative control, A24-C1R cells not pulsed with pep#4 peptide were used. E/T represents a number ratio of CTLs/A24-C1R cells. The results were as shown in Figure 2. As shown in Figure 2, CTLs exhibited antigen-specific cytotoxic action on A24-C1R cells pulsed with pep#4 peptide. From this, it was found that pep#4 peptide induces antigen-specific CTLs.

Example 2: Synthesis of a peptide of a 15 amino acid length and T cell induction

Four types of peptides having a 15 amino acid length with #4 peptide (9 amino acid length) shown in Table 1 at the center were synthesized. The amino acid sequences of the four types of peptides were as follows.

[Formula 1]

```
        LQELGKYEQYIKWPWYIWLGFIAGLIA
15mer-1 LQELGKYEQYIKWPW
15mer-2     GKYEQYIKWPWYIWL
15mer-3         QYIKWPWYIWLGFIA
15mer-4             WPWYIWLGFIAGLIA
```

As mentioned above, 15mer-1 peptide is a partial peptide sequence of 15mer prepared by deleting two amino acids from the C terminal of a sequence of the 9 amino acids and adding 8 amino acids to the N terminal. Peptides (15mer-2 to -4) are partial peptide sequences of 15mer, each involving a shift of four amino acids toward the C terminal to another peptide.

[Table 2]

Table 2: Positions of four peptides having a 15 amino acid length

| | Amino acid sequence (SEQ ID NO) | Position |
|---|---|---|
| 15mer-1 | LQELGKYEQYIKWPW (SEQ ID NO: 9) | 1200-1214 |
| 15mer-2 | GKYEQYIKWPWYIWL (SEQ ID NO: 10) | 1204-1218 |
| 15mer-3 | QYIKWPWYIWLGFIA (SEQ ID NO: 11) | 1208-1222 |
| 15mer-4 | WPWYIWLGFIAGLIA (SEQ ID NO: 12) | 1212-1226 |
| *The number listed in the column of "position" represents the position in the amino acid sequence represented by SEQ ID NO: 8 | | |

Culture was carried out for 21 days in presence of IL-2 and a peptide and stimulation with a peptide was carried out in the presence of brefeldin and monensin in the same manner as in Example 1, and then, INF-γ production was examined. More specifically, to PBMCs of 4 healthy volunteers (HV) with HLA-A2402, a mixture of 15mer peptides was added such that the concentration of each peptide became 10 μg/ml. The PBMCs were cultured in the presence of IL-2. As a result, T cells having a binding affinity to a peptide in contact with them survive. Autogenous PBMCs were pulsed with a mixture of 15mer peptides every week, irradiated with 40 Gy and added to the above culture as a feeder. On Day 14 and 21, the mixture of 15mer peptides was further added. Thereafter, the culture obtained was pulsed with individual 15mer peptides and cultured for 16 hours in the presence of brefeldin and monensin to induce INF-γ production. Intracellular cytokine staining (anti-IFN-g-APC, anti-TNF-a-PE) were performed and peptide-specific cytokine producing CD8T cells were analyzed.

The results were as shown in Figures 3 and 4. As shown in Figure 3, in response to restimulation with 15mer-2, 15mer-3 and 15mer-4, INF-γ production and TNF-α production were increased by CD8 single-positive T cells. This means that the cytotoxicity of T cells is increased by the peptides. Figure 4 is a graph formed by plotting a ratio of INF-γ-positive, TNF-α-positive CD8T cells selected from the cells shown in Figure 3. Of 15mer peptides, a 15mer-3 peptide stimulated T cells most satisfactorily.

Subsequently, TCR repertoire that resultant T cells have was analyzed.

CTL strain, which was established by restimulating the strain with peptide# 4 or a 15mer peptide of Table 1 twice or three times and with autologous PBMCs used as a feeder, was cultured for 6 hours in the presence or absence of peptide# 4 (10 μM) shown in Table 1 after anti-human CD107a-BV421 was added, stained with anti-human CD8-PE and Aqua, and subjected to plate sorting by use of FACS Aria. In the plate sorting, peptide-specific CD8+CD107a+ cells were sorted to be present at a density of 1 cell/well of a 96-well round bottom plate. With respect to a 15mer peptide, anti-human CD107a-BV421 was added and cultured in the presence or absence of a peptide mixture (each peptide 10 μg/ml) for 16 hours, stained with anti-human CD8-PE and Aqua, subjected to plate sorting by use of FACS Aria. In the plate sorting, peptide-specific CD8+CD107a+ cells were sorted to be present at a density of 1 cell/well of a 96-well round bottom plate. TCR of T cells in each cell was analyzed. The results were as shown in Tables 3 and 4.

[Table 3]

Table 3: TCR repertoire of T cells obtained by stimulation with 9mer peptide #4 shown in Table 1

| | TRA | TRB |
|---|---|---|
| 1 | TRAV12-2.02, TRAJ22.01 | TRBV6-6.01, TR8J1-5.01, TRBD1.01 |
| 2 | TRAV23/DV6.01, TRAJ8.01 | TRBV6-6.01, TRBJ1-5.01, TRBD1.01 |
| 3 | TRAV10.01, TRAJ18.01 | TRBV6-6.01, TRBJ1-5.01, TRBD1.01 |
| 4 | TRAV16.01, TRAJ40.01 | TRBV27.01, TRBJ2-3.01, TRBD2.02 |
| 5 | TRAV16.01, TRAJ40.01 | TRBV25-1.01, TRBJ1-4.01, TRBD2.01 |
| 6 | TRAV22.01, TRAJ48.01 | TRBV12-4.01 or TRBV12-4.02, TRBJ2-1.01, TRBD2.01 |

(continued)

| | TRA | TRB |
|---|---|---|
| 7 | TRAV22.01, TRAJ48.01 | TRBV25-1.01, TRBJ1-4.01, TRBD2.01 |
| 8 | TRAV10.01, TRAJ18.01 | TRBV25-1.01, TRBJ1-4.01, TRBD2.01 |
| 9 | TRAV12-2.02, TRAJTRAJ40.01 | TRBV28.01, TRBJ1-3.01, TRBD1.01 |
| 10 | TRAV8-2.03, TRAJ3.01 | TRBV5-6.01, TRBJ2-3.01, TRBD1.01 |
| 11 | TRAV13-1.01, TRAJ43.01 | TRBV20-1.01, TRBJ2-7.01, TRBD2.01 |
| 12 | TRAV10.01, TRAJ18.01 | TRBV25-1.01, TRBJ1-4.01, TRBD2.01 |

[Table 4]

Table 4: TCR repertoire of T cells obtained by stimulation with 15mer peptide mixture

| | TRA | TRB |
|---|---|---|
| 1 | TRAV23/DV6.01, TRAJ8.01 | TRBV6-6.01, TRBJ1-5.01, TRBD1.01 |
| 2 | TRAV12-2.02, TRAJ43.01 | TRBV7-3.01, TRBJ2-3 .01, TRBD2.02 |
| 3 | TRAV26-1.01, TRAJ3.01 | TRBV12-3.01, TRBJ2-1.01, TRBD2.01 |
| 4 | TRAV14/D4.03, TRAJ28.01 | TRBV7-9.03, TRBJ2-1.01, TRBD1.01 |
| 5 | TRAV8-6.02, TRAJ17.01 | TRBV11-2.01, TRBJ2-7.01, TRBD2.01 |
| 6 | TRAV13-1.01, TRAJ30.01 | TRBV5-1.01, TRBJ1-1.01, TRBD2.01 |
| 7 | TRAV8-6.01, TRAJ42.01 | TRBV4-1.01, TRBJ1-2.01, TRBD1.01 |
| 8 | TRAV14/D4.01, TRAJ24.03 | TRBV28.01, TRBJ2-3.01, TRBD2.01 |
| 9 | TRAV25.01, TRAJ50.01 | TRBV25-1.01, TRBJ1-2.01. TRBD1.01 |
| 10 | TRAV27.01, TRAJ21.01 | TRBV6- 5.01, TRBJ1-3.01, TRBD2.01 |
| 11 | TRAV17.01, TRAJ44.01 | TRBV7-8.01, TRBJ2-7.01, TRBD1.01 |
| 12 | TRAV17.01, TRAJ43.01 | TRBV7-8.01, TRBJ2-7.01, TRBD1.01 |
| 13 | TRAV29/DV5.01, TRAJ37.02 | TRBV19.01, TRBJ2-7.01, TRBD2.02 |
| 14 | TRAV10.01, TRAJ18.01 | TRBV25-1.01, TRBJ1-4.01, TRBD2.01 |
| 15 | TRAV22.01, TRAJ48.01 | TRBV25-1.01, TRBJ1-4.01, TRBD2.01 |
| 16 | TRAV8-4.03, TRAJ20.01 | TRBV5-6.01, TRBJ2-5.01, TRBD1.01 |
| 17 | TRAV8-4.03, TRAJ9.01 | TRBV27.01, TRBJ2-3.01, TRBD1.01 |
| 18 | TRAV13-1.02, TRAJ36.01 | TRBV7-9.03, TRBJ2-7.01, TRBD2.01 |
| 19 | TRAV10.01, TRAJ18.01 | TRBV25-1.01, TRBJ1-4.01, TRBD2.01 |
| 20 | TRAV10.01, TRAJ18.01 | TRBV25-1.01, TRBJ1-4.01, TRBD2.01 |
| 21 | TRAV41.01, TRAJ57.01 | TRBV28.01, TRBJ2-3.01, TRBD2.01, |

As shown in Tables 3 and 4, TCR $\alpha$ and $\beta$ genes of viable T cells obtained by stimulation with a 9mer peptide exhibited 12 types of variations, whereas TCR $\alpha$ and $\beta$ genes of viable T cells obtained by stimulation with a 15mer peptide mixture exhibited 21 types of variations. As estimated, it was demonstrated that as the degree of epitope variation increases, the degree of variation of TCR of T cells to be activated increases. This suggests that the peptide mixture induces T cells having a wide variety of antigen specificities, with the result that T cells having a wide variety of antigen specificities can attack a foreign enemy such as a virus. In other words, it is suggested that even if a virus is partially mutated, the T cells having a wider variety of specificities can subsequently attack the virus. It is also suggested, based

on the above data of TCR, that TCR transgenic cells (T cell, NK cell, NKT cell, γδ T cell, MAIT cell) can be used as a therapeutic drug that can kill SARS-CoV-2 infected cells.

Theoretically, a 15mer peptide can be presented by both HLA class I and class II to induce activation of CD4 single-positive T cells and CD8 single-positive T cells. In Examples of the disclosure, CD8 single-positive T cells were checked. As a result, 3 of the 15mer peptides successfully activated cytokine production by the CD8 single-positive T cells. From this, it is expected that a 15mer peptide similarly activates CD4 single-positive T cells.

The 15mer peptide is taken in a cell, fragmented and presented as an about 9mer partial peptide by HLA. At this time, it is considered that a possible production pattern of a 9mer partial peptide varies, with the result that the peptide acts on TCRs having a higher degree of variation to induce activation of T cells.

## Claims

**1.** A partial peptide of SARS-CoV-2 spike protein comprising an amino acid sequence set forth in SEQ ID NO: 4 or a partial peptide of a spike protein positioned in correspondence with the sequence, and having an 8 to 30 amino acid length.

**2.** The peptide according to claim 1, having a consecutive amino acid sequence of an 8 to 20 amino acid length within the region at positions 1204 to 1226 in an amino acid sequence set forth in SEQ ID NO: 8 or an amino acid sequence of a partial peptide of SARS-CoV-2 spike protein positioned in correspondence with the sequence.

**3.** The peptide according to claim 1 or 2, consisting of the amino acid sequence set forth in SEQ ID NO: 4.

**4.** The peptide according to claim 1 or 2, having an amino acid sequence set forth in any one of SEQ ID NOs: 10 to 12 and a 15 to 18 amino acid length.

**5.** An immunogenic composition comprising the peptide according to any one of claims 1 to 4.

**6.** A pharmaceutical composition comprising the peptide according to any one of claims 1 to 4 for use in inducing immunity to SARS-CoV-2.

**7.** A method for inducing immunity to SARS-CoV-2 in a subject, comprising
administering the peptide according to any one of claims 1 to 4 or the composition according to claim 5 or 6 to the subject.

**8.** The method according to claim 7, wherein the subject is a subject infected with SARS-CoV-2.

**9.** The method according to claim 7, wherein the subject is a subject at risk of having been infected with SARS-CoV-2.

**10.** The method according to claim 7, wherein the subject is a subject having at risk of infection with SARS-CoV-2.

Fig. 1

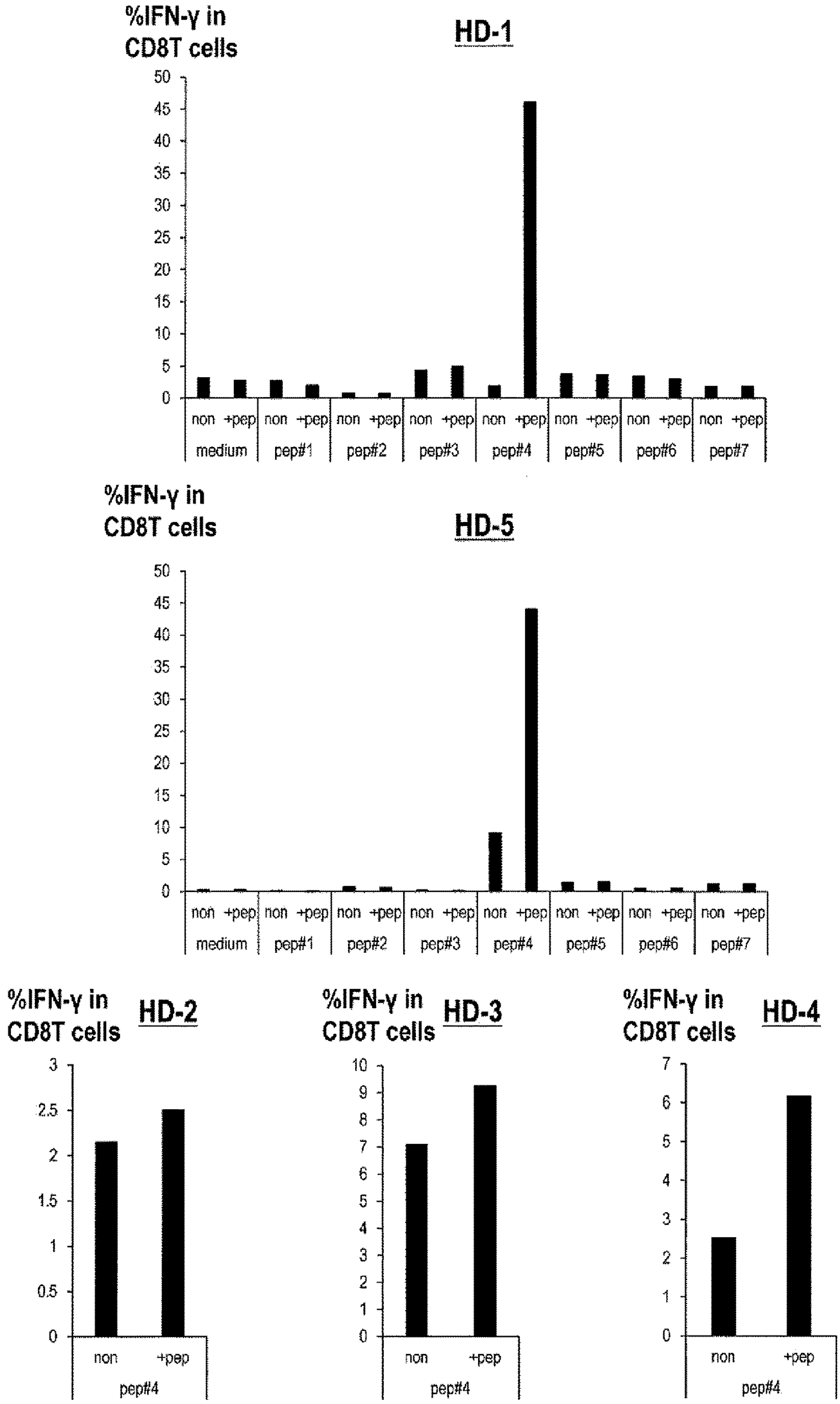

%IFN-γ in CD8T cells
HD-1
50
45
40
35
30
25
20
15
10
5
0
non +pep non +pep non +pep non +pep non +pep non +pep non +pep non +pep
medium pep#1 pep#2 pep#3 pep#4 pep#5 pep#6 pep#7
%IFN-γ in CD8T cells
HD-5
50
45
40
35
30
25
20
15
10
5
0
non +pep non +pep non +pep non +pep non +pep non +pep non +pep non +pep
medium pep#1 pep#2 pep#3 pep#4 pep#5 pep#6 pep#7
%IFN-γ in CD8T cells
HD-2
3
2.5
2
1.5
1
0.5
0
non +pep
pep#4
%IFN-γ in CD8T cells
HD-3
10
9
8
7
6
5
4
3
2
1
0
non +pep
pep#4
%IFN-γ in CD8T cells
HD-4
7
6
5
4
3
2
1
0
non +pep
pep#4

Fig. 2

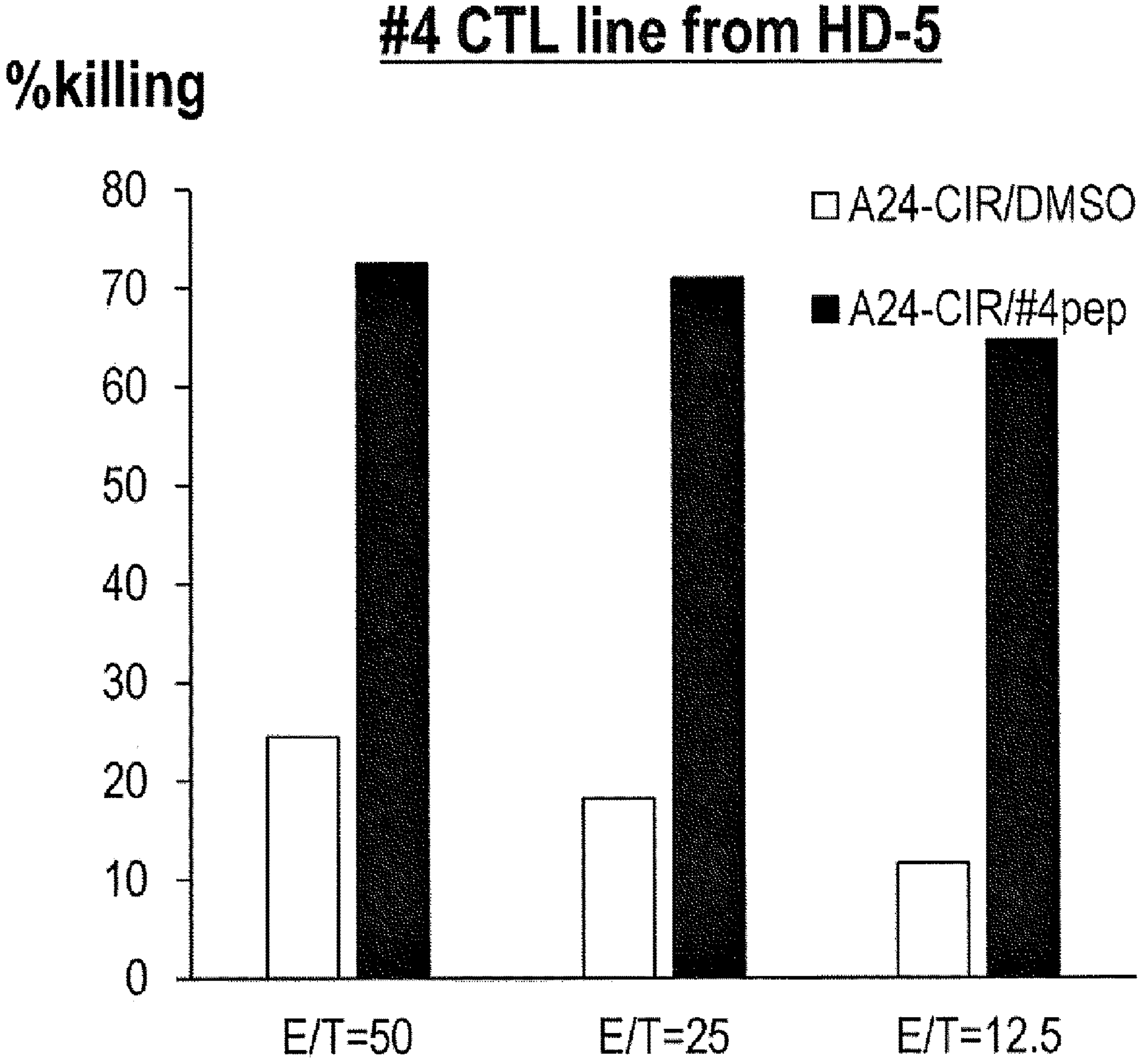
#4 CTL line from HD-5
%killing
80
70
60
50
40
30
20
10
0
A24-CIR/DMSO
A24-CIR/#4pep
E/T=50
E/T=25
E/T=12.5

FIG. 3

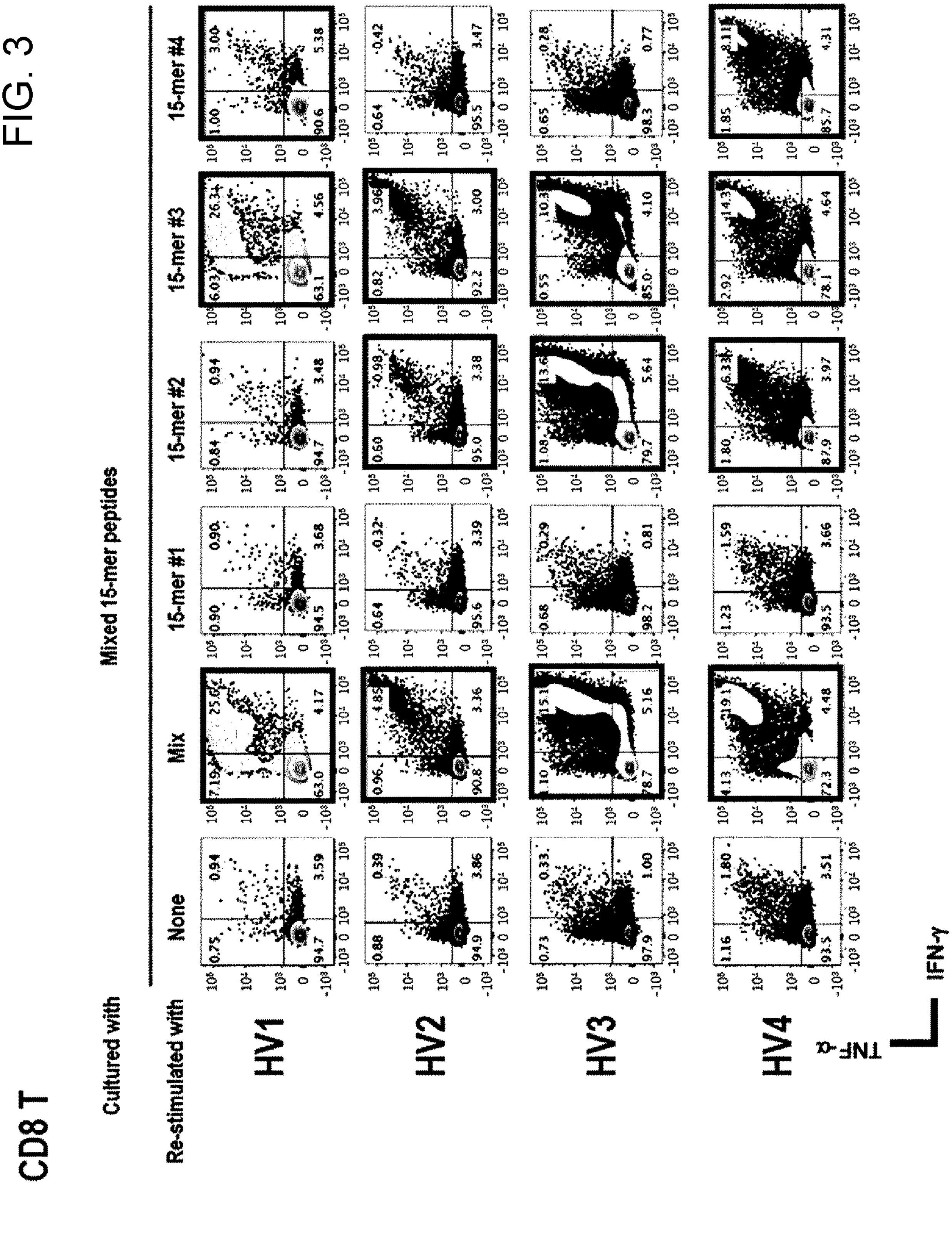
CD8 T
Cultured with
Mixed 15-mer peptides
Re-stimulated with
None
Mix
15-mer #1
15-mer #2
15-mer #3
15-mer #4
HV1
HV2
HV3
HV4
TNF-α
IFN-γ

Fig. 4

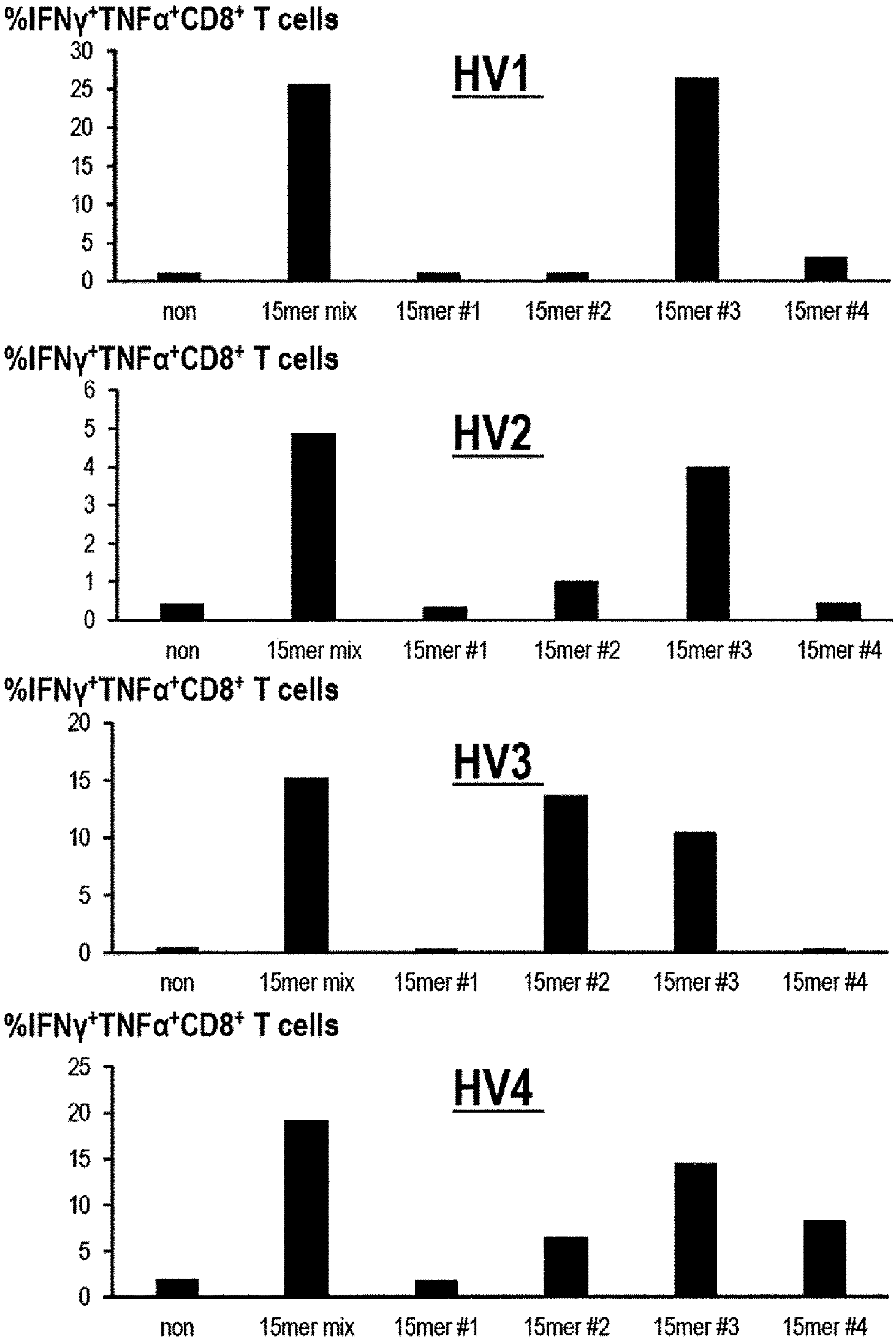
%IFNγ+TNFα+CD8+ T cells
HV1
30
25
20
15
10
5
0
non
15mer mix
15mer #1
15mer #2
15mer #3
15mer #4
%IFNγ+TNFα+CD8+ T cells
HV2
6
5
4
3
2
1
0
non
15mer mix
15mer #1
15mer #2
15mer #3
15mer #4
%IFNγ+TNFα+CD8+ T cells
HV3
20
15
10
5
0
non
15mer mix
15mer #1
15mer #2
15mer #3
15mer #4
%IFNγ+TNFα+CD8+ T cells
HV4
25
20
15
10
5
0
non
15mer mix
15mer #1
15mer #2
15mer #3
15mer #4

**INTERNATIONAL SEARCH REPORT**

International application No. **PCT/JP2021/030565**

**A. CLASSIFICATION OF SUBJECT MATTER**

***C07K 14/165***(2006.01)i; ***A61K 38/08***(2019.01)i; ***A61K 38/10***(2006.01)i; ***A61K 39/215***(2006.01)i; ***A61P 31/14***(2006.01)i; ***C07K 7/06***(2006.01)i; ***C07K 7/08***(2006.01)i; ***C12N 15/50***(2006.01)i
FI: C07K14/165; A61K38/08; A61K38/10; A61K39/215; A61P31/14; C07K7/06; C07K7/08; C12N15/50 ZNA

According to International Patent Classification (IPC) or to both national classification and IPC

**B. FIELDS SEARCHED**

Minimum documentation searched (classification system followed by classification symbols)

C07K14/165; A61K38/08; A61K38/10; A61K39/215; A61P31/14; C07K7/06; C07K7/08; C12N15/50

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY/MEDLINE/EMBASE/BIOSIS (STN); UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| P, X | WO 2021/130377 A1 (THE QUEEN'S UNIVERSITY OF BELFAST) 01 July 2021 (2021-07-01)<br>claim 5, page 3 | 1-10 |
| X | SANTONI, D. et al., "In the search of potential epitopes for Wuhan seafood market pneumonia virus using high order nullomers". Journal of Immunological Methods. [online], 23 April 2020, vol. 481, 482, 112787 (pp. 1-3), Internet <URL:https://www.sciencedirect.com/science/article/abs/pii/S0022175920300661?via%3Dihub>, <doi: 10.1016/j.jim.2020.112787>, [retrieved on: 05 October 2021]<br>table 2 | 1-2, 5-10 |
| Y | | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 October 2021** | **19 October 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2021/030565**

**C. DOCUMENTS CONSIDERED TO BE RELEVANT**

| Category* | Citation of document, with indication, where appropriate, of the relevant passages | Relevant to claim No. |
|---|---|---|
| X<br><br>Y | LI, L. et al., "Epitope-based peptide vaccine design and target site characterization against novel coronavirus dease caused by SARS-CoV-2". bioRxiv. [online], 27 February 2020, 965434, Internet <URL:https://www.biorxiv.org/content/10.1101/2020.02.25.965434v1?versioned=true>, <doi: 10.1101/2020.02.25.965434>, [retrieved on: 05 October 2021]<br>tables 4-6 | 1-2, 4-10<br><br>1-10 |
| X<br><br>Y | SAMAD, A. et al., "Designing a multi-epitope vaccine against SARS-CoV-2: an immunoinformatics approach". Journal of Biomolecular Structure and Dynamics. [online], 17 July 2020, 1792347, Internet <URL:https://www.tandfonline.com/doi/full/10.1080/07391102.2020.1792347>, <doi: 10.1080/07391102.2020.1792347>, [retrieved on: 05 October 2021]<br>tables 2, 4 | 1-2, 4-10<br><br>1-10 |
| Y | SAINZ, B. JR. et al., "The aromatic domain of the coronavirus class I viral fusion protein induces membrane permeabilization: putative role during viral entry". Biochemistry. 23 December 2004, vol. 44, no. 3, pp. 947-958, doi: 10.1021/bi048515g<br>abstract, fig. 1, table 1 | 1-10 |
| Y | LIAO, Y. et al., "Tryptophan-dependent membrane interaction and heteromerization with the internal fusion peptide by the membrane proximal external region of SARS-CoV spike protein" Biochemistry, 10 February 2015, vol. 54, no. 9, pp. 1819-1830, doi: 10.1021/bi501352u<br>abstract, table 2 | 1-10 |
| A | US 2005/0106563 A1 (GENESIS BIOTECH INC.) 19 May 2005 (2005-05-19)<br>GA297 (SEQ ID NO. 193) & GA298 (SEQ ID NO. 194) | 1-10 |

Form PCT/ISA/210 (second sheet) (January 2015)

**INTERNATIONAL SEARCH REPORT**

International application No.
**PCT/JP2021/030565**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/030565**

| Patent document cited in search report | Publication date (day/month/year) | Patent family member(s) | Publication date (day/month/year) |
|---|---|---|---|
| WO 2021/130377 A1 | 01 July 2021 | (Family: none) | |
| US 2005/0106563 A1 | 19 May 2005 | TW 200512215 A<br>GA297 (SEQ ID NO. 193) &<br>GA298 (SEQ ID NO. 194) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GRIFONI et al.** *Cell host & Microbe,* 2020, vol. 27 (4), 671-680